Europäisches Patentamt

(19) European Patent Office

Office européen des brevets

(11) Numéro de publication: **0 022 060**

**B1**

(12) **FASCICULE DE BREVET EUROPEEN**

(45) Date de publication du fascicule du brevet: **19.10.83**

(21) Numéro de dépôt: **80810166.1**

(22) Date de dépôt: **22.05.80**

(51) Int. Cl.³: **G 06 F 15/02, A 61 B 5/00**

(54) **Calculatrice de poche pour le planning familial.**

(30) Priorité: **07.06.79 CH 5336/79**

(43) Date de publication de la demande:
**07.01.81 Bulletin 81/1**

(45) Mention de la délivrance du brevet:
**19.10.83 Bulletin 83/42**

(84) Etats contractants désignés:
**AT BE CH DE FR GB IT LI LU NL SE**

(56) Documents cités:
**FR - A - 2 154 722**
**US - A - 3 884 219**
**US - A - 4 116 228**

**IEEE TRANSACTIONS ON BIO-MEDICAL ENGINEERING BME-12 janvier 1965 NEW YORK (US) A. J. ADDUCCI: "Ovulation detection by internal cranial temperature measurements", pages 2—7.**

(73) Titulaire: **Bioself International Inc.**
**P.O. Box 10496 Shirley Street 50**
**Nassau, Bahamas (BS)**

(72) Inventeur: **Bellet, Jean-Marie**
**c/o E. Desjacques 91, Rue de Genève**
**CH-1226 Thônex GE (CH)**

(74) Mandataire: **Kirker, Gaylord Emile**
**c/o KIRKER & Cie S.A. 14, Rue du Mont-Blanc**
**Case postale 872**
**CH-1211 Genève 1 (CH)**

Courier Press, Leamington Spa, England.

# Calculatrice de poche pour le planning familial

L'invention concerne une calculatrice pour le planning familial.

On sait actuellement réaliser des calculatrices miniatures capables d'effectuer des calculs relativement complexes.

On connaît d'autre part des méthodes de planning familial telles que par exemple la méthode Ogino. La mise au point d'un programme de calcul pour l'application de cette méthode et l'incorporation d'un tel programme à une calculatrice de poche sous la forme notamment d'une ROM (mémoire morte à relecture seulement) n'offre en principe aucune difficulté.

Cependant la calculatrice doit posséder une mémoire vive (RAM) permanente pour les données permettant l'application de la méthode, ces données concernant les dates des périodes cataméniales de la personne intéressée ainsi que le graphe de sa température jour par jour. Ces données doivent être fournies pendant quelques cycles pour que la méthode soit applicable et permette de prévoir les dates du prochain cycle.

La conservation de ces données en mémoire implique l'utilisation de mémoires particulières dont le contenu ne se perd pas lorsque l'alimentation est coupée.

La demande européenne 79 810067.3 (publiée le 28.5.80 sous le numéro 11594) traite de ce problème.

Un autre problème rencontré concerne l'acquisition des données de température. Une solution envisagée consiste à prendre la température à l'aide d'un thermomètre médical à mercure classique et de reporter ensuite le résultat sur le clavier de la calculatrice pour l'inscrire dans la mémoire. Cependant, cette solution présente l'inconvénient d'un risque sensible d'erreur humaine au moment de l'inscription de la donnée sur le clavier. Une autre solution connue, illustrée par les dispositifs décrits dans les documents DE—A—2 803 152, FR—A—2 154 722 et US—A—3 884 219, consiste à utiliser une sonde thermométrique reliée par un câble électrique à un dispositif d'acquisition de données. La présente invention vise à rendre plus aisée l'acquisition d'une donnée de température et se rapporte à une calculatrice de poche prévue pour appliquer une méthode de planning familial et munie d'une sonde thermométrique pour rendre simple et sûre l'acquisition des données de température, cette sonde devant fonctionner sans être reliée par un câble électrique à la calculatrice.

A cette fin, l'invention propose la calculatrice et la sonde définies, respectivement, dans les revendications 1 et 7.

L'invention sera mieux comprise à l'aide de la description de quelques formes d'exécution faites ci'après en référence aux dessins sur lesquels:

La fig. 1 représente une calculatrice de poche pour le planning familial,

la fig. 2 représente une sonde thermométrique destinée à la calculatrice de la fig. 1,

la fig. 3 représente une vue de la face étroite supérieure de la calculatrice,

la fig. 4 représente une configuration de système à microprocesseur pour la calculatrice,

la fig. 5 représente un appareil de contrôle,

la fig. 6a représente une autre forme de réalisation de la calculatrice, et

la fig. 6b est une vue de dos d'une partie de la calculatrice de la fig. 1,

la fig. 7 représente les circuits d'une sonde thermométrique,

la fig. 8 représente de manière schématique les circuits d'une sonde thermométrique, et

la fig. 9 représente une autre forme de réalisation des circuits d'une sonde thermométrique.

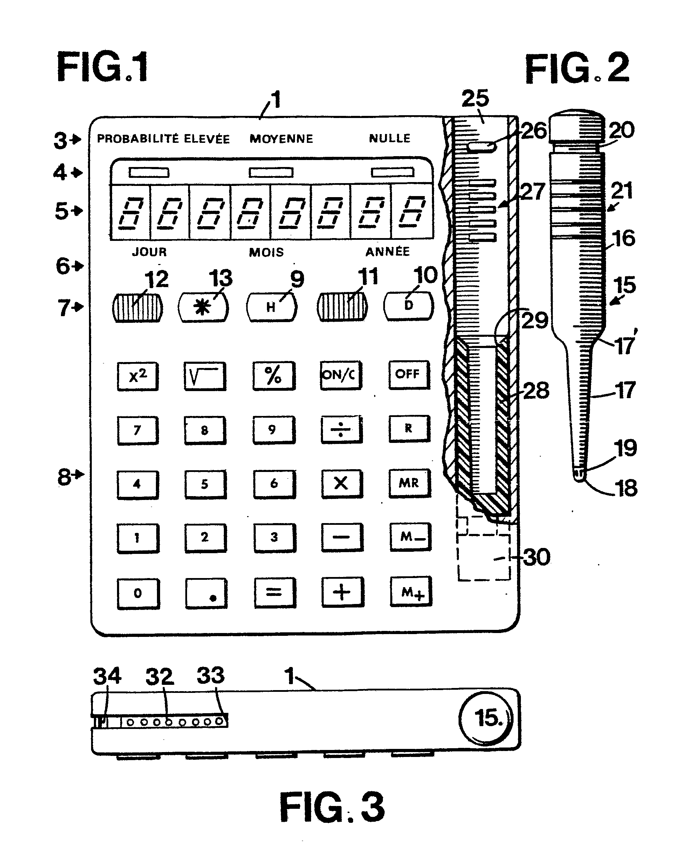

La calculatrice représentée à la fig. 1 comporte un boîtier 1 comprenant les circuits, non représentés, et sur lequel on voit les moyens d'affichage 4, 5, entourés d'inscriptions 3, 6, et des touches de commande 7, 8.

L'affichage numérique 5 et les touches numériques 8 sont semblables aux parties correspondantes d'une calculatrice ordinaire à quatre opérations. La partie supérieure 4 de l'affichage comporte trois zones disposées chacune en regard d'une inscription de la ligne 3 d'inscription. Cette partie 4 de l'affichage est destinée à indiquer le résultat d'un calcul de prévision appliquant une méthode de planning familial. L'affichage indique la probabilité pour qu'un rapport intime, à une date donnée, soit suivi d'une grossesse. L'affichage 4 permet d'indiquer si la probabilité est élevée, moyenne ou nulle.

La partie numérique 5 de l'affichage permet d'afficher les données et les résultats arithmétiques liés à l'utilisation comme calculatrice ordinaire à quatre opérations et à la manipulation du clavier numérique 8. Cependant, cet affichage numérique 5 permet également d'afficher une date, les deux digits de gauche donnant le jour, le digit suivant étant non activé, les deux digits suivants donnant le mois, le digit d'après étant non activé, et les deux derniers digits indiquant l'année. Cette partie numérique permet également d'afficher l'heure présente, un clignotement ou le défilement des secondes permettant de distinguer l'affichage de l'heure de l'affichage d'une date.

Parmi les touches de la rangée 7, la touche 9 ''H'' permet de commander l'affichage de l'heure et les autres touches 10 à 13 concernent plus spécifiquement l'utilisation de la calculatrice pour l'application d'une méthode de planning familial. La touche 10 ''D'' commande l'affichage de la date et permet également l'inscription d'une autre date, pour laquelle on ordonne l'exécution d'un calcul de prévision. Les touches 11 et 12 permettent de noter en mémoire la date des règles.

Si cette date est celle du jour même, on peut la faire apparaître sur l'affichage 5 par simple appel au moyen de la touche "D". Si c'est une autre date, on peut introduire cette autre date à l'aide du clavier numérique en pressant D après les chiffres du mois, les chiffres du jour et les chiffres de l'année. La date est affichée. On enfonce alors simultanément les touches 11 et 12 et la date est alors enregistrée en tant que date du début des règles. Cette opération modifie les données; c'est pourquoi il a été prévu que son inscription nécessite l'action simultanée sur deux touches pour réduire les risques d'une inadvertance. La date est affichée selon le mode français, c'est-à-dire jour, mois, an, en correspondance avec les inscriptions 5. Mais il est évident que l'on peut prévoir d'autres modes.

La touche 13 "*" permet de commander le calcul d'une prévision pour la date affichée sur l'affichage numérique 5, le résultat apparaissant sur l'affichage supérieur 4; une double action sur la même touche "*" permet de commander le calcul d'une prévision donnant l'intervalle autour d'une ovulation pendant lequel les probabilités d'une grossesse sont maximales. Les deux dates extrêmes sont affichées alternativement avec une période d'une à deux secondes.

La calculatrice comporte un logement 25 pour la sonde thermométrique 15 de la fig. 2. Le logement comporte un ressort de retenue 26 destiné à coopérer avec une rainure annulaire de retenue 20 ménagée sur le pourtour du corps 16 de la sonde 15. Le logement 25 comporte aussi des contacts 27 destinés à coopérer avec des contacts annulaires 21 disposés sur le corps de la sonde. Le fond du logement 25 est occupé par une douille mobile axialement 28 comportant une ouverture conique 29, et destinée à recevoir la partie mince 17 de la sonde 15 au bout de laquelle se trouve le capteur 18 de la sonde. On remarque que la longueur de la cavité de la douille est supérieure à la longueur de la partie mince 17 de la sonde afin que le capteur 18 ne touche pas le fond de la cavité, la sonde étant retenue par l'épaulement 17' au niveau de l'ouverture conique 29. On remarque également que le corps 16 est cylindrique et plus long que la partie mince, de manière que lorsqu'on introduit la sonde dans son logement, la partie mince se présente en regard de l'ouverture conique sans buter sur les bords de cette ouverture. La douille 28 est mobile axialement et coopère avec un mécanisme à cliquet et à ressort 30 analogue à celui existant dans un stylo bille et agencé de telle sorte que lorsque le capteur est en place et qu'on l'enfonce d'environ 2 mm dans son logement, le mécanisme 30 réagisse en repoussant la douille et donc la sonde, de manière qu'on puisse la saisir et l'extraire de son logement en vue d'une utilisation. Lorsque la sonde est au repos dans son logement, elle affleure juste la surface du boîtier. Sur la sonde elle-même on remarque également un capteur d'humidité 19 permettant de détecter le contact d'une muqueuse.

La face supérieure de la calculatrice, représentée à la fig. 3, montre l'extrémité de la sonde 15, et montre aussi un connecteur extérieur 32 à plusieurs contacts, disposé dans une cavité 33. La cavité est en principe masquée par un couvercle comportant une languette de retenue coopérant avec une rainure de retenue 34.

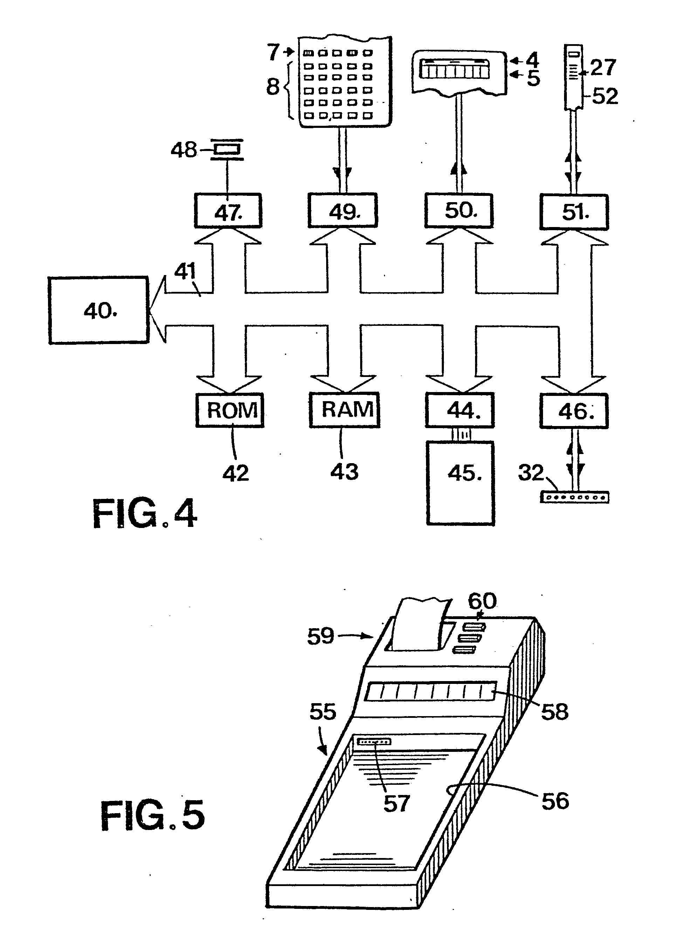

La calculatrice présente une configuration de système à microprocesseur représentée à la fig. 4, dont certaines parties sont classiques. Le système comporte un processeur 40 relié par un bus 41 à des mémoires et à des périphériques. Parmi les mémoires on trouve une mémoire ROM 42 pour les programmes et les données fixed de la mémoire RAM 43, une mémoire spéciale 45 à relais électromécaniques bistables, avec son interface 44. On trouve un exemple d'une telle mémoire dans la demande suisse 8455/78 précédemment mentionnée.

Les relais utilisés sont des relais miniaturisés présentant une grande immunité aux accélérations et à divers environnements et, surtout, conservant l'information mémorisée même si l'alimentation est coupée.

Le système représenté comprend encore une unité de mesure du temps 47 fonctionnant avec un quartz 48. Le système comprend les périphériques constitués par le clavier comprenant les touches 7, 8, et les dispositifs d'affichage 4, 5, par exemple du type LCD (à cristaux liquides), avec leurs interfaces respectifs 49, 50. Le système comprend enfin le connecteur de sonde 52 incluant les contacts 27 du logement de sonde, avec l'interface 51, et le connecteur extérieur 32 avec son interface 46.

L'appareil de la fig. 5 est destiné à effectuer certaines opérations de contrôle sur la calculatrice. En principe un tel appareil est destiné à un gynécologue ou à une personne compétente d'un centre de planning familial. Lorsqu'une personne acquiert une calculatrice, l'initialisation nécessite certaines opérations de mise en route qu'il n'est pas indiqué de laisser faire à la personne intéressée, car le but de la calculatrice est de rendre simple l'application d'une méthode, alors que les opérations de mise en route sont un peu délicates. D'autre part, l'appareil permet d'accéder au contenu mémorisé, pour établir par exemple le graphe des températures au jour le jour, ce qui fournit au gynécologue une indication utile pour une consultation. Une des opérations de mise en route concerne la mise à l'heure et à la date après que la calculatrice a été munie de ses batteries d'alimentation. Il est préférable que la personne utilisant la calculatrice ne puisse pas, en principe, procéder elle-même à cette opération qui est faite une seule fois pour la durée de service des batteries; en effet, si la remise à l'heure était possible en utilisation normale, il se pourrait que par inadvertance la personne altère les données en introduisant par erreur une date erronée, en suite de quoi les prévisions seraient faussées.

L'appareil 55 comporte un logement 56 destiné à recevoir une calculatrice du type précédemment décrit. Dans le logement, un connecteur 57 est prévu pour coopérer avec le connecteur externe 32 de la calculatrice. L'appareil comporte un affichage 58, des touches de commande 60 et une imprimante 59 permettant par exemple d'établir le graphique des températures jour par jour à partir des données mémorisées dans la calculatrice. L'appareil permet également la mise à l'heure et à la date de la calculatrice, par exemple selon le principe suivant.

Le système comporte un programme fixe en ROM permettant d'imposer un contenu d'heure et de date à l'unité de mesure du temps, cela à partir d'ordres donnés depuis le clavier numérique 8, mais uniquement à la condition que la calculatrice soit connectée à un appareil de contrôle 55, et que l'on ait actionné une touche adéquate sur le dit appareil de contrôle, cette touche agissant sur le système à microprocesseur via le connecteur externe 32 et son interface 46.

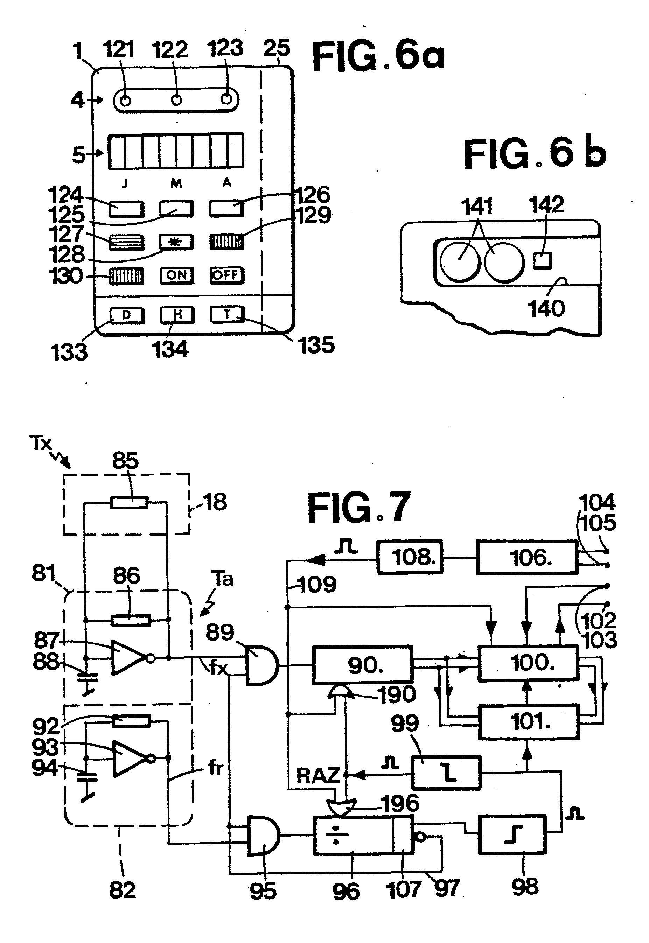

La fig. 6a illustre une autre forme de réalisation de la calculatrice, se distinguant par un clavier plus simple et qui n'est pas destinée à être connectée à un appareil de contrôle.

Sur le boîtier 1 on distingue un affichage spécial 4 formé de trois éléments électro-optiques 121, 122, 123, respectivement rouge, jaune et vert, destiné à indiquer le résultat d'un calcul de prévision selon la convention suivante: probabilité élevée, rouge; moyenne, jaune; nulle, vert. L'affichage numérique 5 permet d'afficher l'heure, une date ou une température. La touche "H" 134 permet de commander l'affichage de l'heure présente et la touche "D" 133 permet de commander l'affichage de la date actuelle, la touche "*" 128 permettant de commander un calcul de prévision pour cette date ou, par double action, le calcul de l'intervalle autour de l'ovulation où la probabilité d'une grossesse est maximale. Les touches "J", "M" et "A" 124, 125 et 126 permettent de changer la date affichée. Une action sur l'une des touches fait augmenter le chiffre correspondant, soit le jour pour la touche "J", le mois pour la touche "M" et l'année pour la touche "A". La touche 127 permet d'inverser le sens de l'action des touches 124 à 126 qui, alors, font diminuer les chiffres respectifs. Les touches 129, 130 permettent l'inscription de la date affichée comme date du premier jour des règles.

La fig. 6b montre partiellement le dos de la calculatrice avec le logement 140 pour les piles 141, ce logement comprenant également une touche spéciale de commande 142. Cette touche 142 est normalement inaccessible, étant masquée par le couvercle du logement des piles. Cette touche est destinée à permettre certaines opérations de contrôle ou de réglage effectuées en principe par une personne spécialement formée d'un centre de planning familial, ou le gynécologue de la personne intéressée.

Une action sur cette touche permet d'activer un programme prévu pour la mise à l'heure et à la date de la calculatrice, lors de sa mise en fonction, ainsi que l'accès à la mémoire, au moins pour y lire les contenus mémorisés qui apparaissent sur l'affichage 5 par exemple selon la procédure et suivant le format décrit ci-après.

On tient dans une main la calculatrice, le logement de pile 140 étant découvert, ce qui donne accès à la touche 142 à l'une des doigts de la main tenant la calculatrice. L'autre main reste libre pour agir sur le clavier. Tout en maintenant actionnée la touche de commande 142, on actionne la touche "T" 135 et l'affichage 5 montre alors la température mémorisée la plus ancienne, alternativement avec sa date, et lorsqu'on appuie à nouveau sur "T", l'affichage montre la température du jour suivant alternativement avec sa date, et ainsi de suite, ce qui permet d'établir le graphe de la température. De plus, pour le jour des régles, la lettre "r" est affichée avec la température, et pour les dates d'un intervalle autour de l'ovulation, l'élément lumineux 121 est allumé. Pour l'inscription de la date et de l'heure, on actionne les touches 124 à 127 pour faire apparaître la date actuelle et on l'inscrit par action simultanée de la touche "D" 133 et de la touche d'inscription 129, la touche de commande 142 étant maintenue pressée. Pour inscrire l'heure, on actionne la touche "H" 134 puis au besoin on modifie l'heure affichée au moyen des touches 124 à 127 et on l'inscrit par action simultanée sur la touche "H" et sur la touche d'inscription 129, la touche de commande 142 étant maintenue pressée.

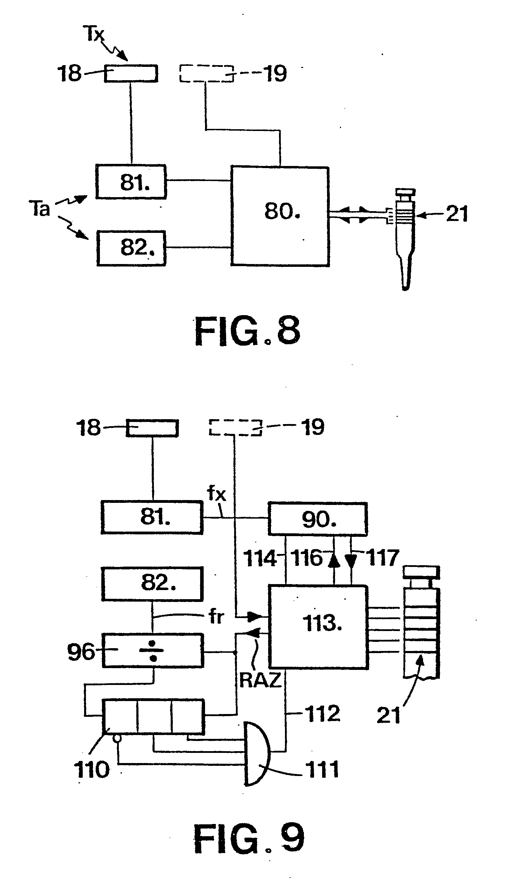

La fig. 7 montre un exemple de schéma électrique pour une sonde thermométrique destinée à une calculatrice de poche pour le planning familial. Dans ce circuit, le capteur 18 est, lors de la mesure, porté à la température à mesurer $T_x$. Le capteur est ici formé d'une résistance NTC 85, par exemple de 600 kΩ, avec une caractéristique de 5 %/°, soit 30 kΩ/° autour de sa valeur nominale. Cette NTC est reliée au circuit d'un oscillateur à relaxation 81 formé d'un trigger inverseur 87 et d'un réseau RC comprenant une capacité 88 et une résistance 86 en parallèle avec la NTC 85. La résistance 86 n'est pas indispensable, notamment dans le cas où la résistance NTC 85 possède une caractéristique bien linéaire dans le domaine utile. L'oscillateur 81 délivre des impulsions logiques à fréquence $f_x$ dépendant de la température $T_x$. Ces impulsions passent, via une porte ET 89 dans un compteur 90.

Le circuit comporte un oscillateur de référence 82 de construction semblable au premier oscillateur et voisin de lui. Cet oscillateur comporte un trigger inverseur 93 et un réseau RC à capacité 94 et résistance 92. L'oscillateur de référence 82 a une fréquence $f_r$ du même ordre de grandeur que $f_x$, par exemple $f_r$ étant la moyenne géométrique entre le maximum et le minimum du domaine utile de $f_x$. Cette fréquence $f_r$ est amenée, via une porte ET 95 à un diviseur 96 qui la divise par un nombre représentant l'inverse de la précision relative recherchée pour la mesure. Par exemple on peut supposer que $f_x$ est de l'order de 100'000 Hz, $f_r$ étant pris égal à 100'000 Hz, et que l'on désire une précision de 1 millionième pour le comptage de la fréquence $f_x$, l'étage diviseur étant alors un diviseur de fréquence par un facteur de 1 million, ou voisin d'un million, par exemple $2^{20}$; $(2^{10}=1024)$. Le diviseur 96 se termine par une bascule 107 dont la sortie inversée commande les portes ET 89 et 95, et dont la sortie non inversée commande deux bascules monostables successives 98 et 99, la première servant à l'inscription du résultat du comptage et la seconde servant à la remise à zéro. La sortie de la seconde bascule 99 aboutit à des portes OU 190 et 196 qui commandent les entrées de mise à zéro du compteur 90 et du diviseur 96 respectivement. Le compteur 90 est relié à une mémoire 100 et à une première entrée d'un comparateur 101, la seconde entrée de ce comparateur étant reliée à la mémoire 100. Pour transférer le contenu de la mémoire vers le système, la mémoire 100 comporte une ligne de sortie "série" et une ligne de synchronisation pour le transfert "série" reliées respectivement aux bornes 103 et 102. Le circuit possède encore un bloc de commande et d'alimentation 106 relié aux bornes 104, 105, les bornes 102 à 105 aboutissant aux contacts annulaires 21 de la sonde.

En plus de sa fonction d'alimentation électrique le bloc 106 possède une fonction de mise à zéro initiale du compteur 90, du diviseur 96 et de la mémoire 100. A cette fin, le bloc 106 commande une bascule monostable 108 dont la sortie est reliée à une entrée de mise à zéro de la mémoire 100 et aux portes OU 190 et 196 commandant l'entrée de remise à zéro du compteur 90 et du diviseur 96 respectivement.

Le fonctionnement de ce circuit est le suivant.

Lorsque la sonde est connectée, les bornes 104 et 105 permettent le chargement de la batterie d'alimentation à partir de la calculatrice, le circuit 106 étant agencé pour mettre hors fonction les autres éléments du circuit de la fig. 7 tant que la sonde est connectée à la calculatrice. Lorsqu'on retire la sonde en vue d'une prise de température, ses circuits sont mis en fonction, le compteur, le diviseur et la mémoire étant initialisés à zéro. Dès que la sonde est retirée, les deux circuits oscillants délivrent respectivement leurs fréquences $f_x$ et $f_r$. Lorsque le diviseur 96 a accompli un cycle, son étage final 107 se met à 1 et la ligne 97 inhibe les portes 89 et 95, ce qui bloque le comptage. En même temps, le monostable 98 envoie une impulsion de commande mettant en action le comparateur 101. Si le contenu du compteur 90 est supérieur au contenu de la mémoire 100, ce contenu est inscrit dans la mémoire 100. A la fin de l'impulsion du monostable 98, un monostable 99 envoie une impulsion de remose à zéro du compteur 90 et du diviseur 96, y compris son étage terminal 97, ce qui active à nouveau les portes 89 et 95, de sorte qu'un nouveau cycle de mesure commence.

Si la mesure du nouveau cycle donne un résultat supérieur, c'est-à-dire si la température $T_x$ a augmenté, alors l'action du comparateur a pour effet que c'est cette dernière valeur qui est enregistrée. La sonde fonctionne ainsi comme un thermomètre à maximum. L'avantage de cette disposition est le suivant: lorsque la personne retire la sonde après avoir pris sa température et que par conséquent la température du capteur revient à la température de l'air ambient, la température rectale, plus élevée, reste enregistrée. Lorsque la sonde est replacée sur la calculatrice, le système à microprocesseur peut lire le résultat mémorisé via les bornes 102 et 103. La borne 102 transmet des impulsions de synchronisation et la borne 103 transmet en séquence les valeurs binaires représentant le contenu de la mémoire 100.

Il y a lieu de remarquer que l'oscillateur de référence 82 est voisin de l'oscillateur de mesure 81 et assez semblable à lui, à l'exception de l'élément NTC 85 évidemment. De plus, ils reçoivent leur alimentation de la même source. Etant voisins, ils sont en principe à la même température $T_a$. Par conséquent, si la fréquence $f_x$ du premier oscillateur, fonction de la température $T_x$, est en outre influencée par une dérive thermique ou une dérive d'alimentation agissant sur le circuit 81, on peut s'attendre à ce qui la fréquence de référence $f_r$ soit affectée dans le même sens et par la même dérive agissant sur l'oscillateur de référence 82. Dès lors, si une telle dérive ralentit par exemple $f_x$, elle ralentira $f_r$ dans la même proportion, ce qui allongera la durée du comptage, de sorte que finalement l'effet de la dérive sera annulé.

La partie logique du circuit de la fig. 7 est construite à partir d'éléments relativement simples tels que compteurs, portes, registres. Toutefois, pour les fonctions du circuit de la fig. 7, ou a fortiori pour des fonctions plus complexes, on peut préférer faire d'emblée appel à un microprocesseur. Dans le circuit de la fig. 8, on retrouve un oscillateur de mesure 81 relié à un capteur 18, et un oscillateur de référence 82, qui sont reliés à un circuit de commande 80 qui peut être à microprocesseur. Le circuit est relié à un connecteur aboutissant aux contacts annulaires 21 de la sonde thermométrique.

Par ailleurs, le circuit de la fig. 8 peut comporter un détecteur d'humidité 19. La température à mesurer est en principe la température rectale et le but du détecteur d'humidité est de fournir une indication du contact avec la paroi du rectum, suffisamment humide pour faire réagir un détecteur approprié. Le signal fournit donc une indication supplémentaire quant à la validité de la mesure. Suivant la méthode classique, la température doit être prise au réveil. On peut par conséquent utiliser l'heure comme condition pour une mesure valide. Cette condition est évidemment verifiée par le système de la calculatrice, qui consulte son unité de mesure du temps au moment où la sonde est replacée après avoir été sortie. On peut agencer le système par exemple de manière qu'il considère comme non valide une mesure de la température effectuée dans la même journée qu'une première mesure, ou à une heure trop écartée par rapport à l'heure habituelle de réveil, ou aussi une température en dehors du domaine des températures physiologiques. Le programme peut aussi être raffiné au point de faire la différence entre le changement de température lié au cycle cataménial, et un changement de température momentané d'origine pathologique tel qu'une légère fièvre ou une légère hypothermie. Il est évident qu'une maladie peut rendre la méthode inapplicable. Ces critères sont appliqués par le système de la calculatrice.

Une autre condition de validité de la mesure est le fait que la sonde soit en équilibre thermique avec la zone à mesurer. On peut à cette fin prévoir sur la sonde elle-même des moyens de temporisation.

La fig. 9 représente un circuit de sonde muni d'un moyen de temporisation. Le circuit comporte un capteur 18 relié à un oscillateur de mesure 81 dont la sortie est reliée à un compteur 90, et un oscillateur de référence 82 dont la sortie est reliée à un diviseur de fréquence 96. Le diviseur 96 est suivi d'un diviseur 110 à trois étages binaires. La sortie inversée du premier de ces étages et les sorties normales des deux autres sont reliées aux entrées d'une porte ET multiple 111 dont la sortie 112 aboutit à un circuit de commande 113. Ce circuit commande le compteur 90 via la ligne 114. Le compteur 90 est en outre agencé pour pouvoir être lu en mode série à l'aide d'une ligne de lecture 117 et d'une ligne de synchronisation 116.

Le fonctionnement est le suivant. Initialement, le compteur 90 et les diviseurs 96 et 110 sont à zéro. La période à la sortie du diviseur 96 est supposée, à titre d'exemple, égale à 10 secondes. La sortie 112 de la porte 11 est dans l'état "1" lorsque le compteur 110 est dans l'état "6" ou "1 1 0" en binaire, cet état ayant une durée de 10 secondes et prenant place entre la 60ème et la 70ème seconde après le début du fonctionnement.

Le circuit 113 est agencé pour actionner le compteur 90, via la ligne 114, exactement pendant le temps où la ligne 112 est à "1", après quoi il met hors fonction les oscillateurs; lorsque la sonde est remise dans son logement, le circuit 113 gère le transfert du nombre compté par le compteur 90 vers le système de la calculatrice. Dans ce circuit on utilise l'oscillateur de référence 82 non seulement comme base de temps pour la durée du comptage, qui permet la mesure de $f_x$ par rapport à $f_r$, mais également comme base de temps pour une temporisation.

## Revendications

1. Calculatrice de poche pour le planning familial, comprenant un boîtier (1), une sonde thermométrique (15), un clavier de commande (7, 8), un affichage (4, 5), des moyens de calcul et de commande électroniques (40) et des mémoires (42, 43, 45) agencés pour l'application d'une méthode de planning familial.

caractérisée en ce qu'elle comporte un logement (25) pour recevoir temporairement la sonde thermométrique (15), en ce que la sonde thermométrique est électriquement indépendante de la calculatrice, en ce que la sonde comporte à une extrémité un capteur thermométrique (18) destiné à être amené en contact avec la zone dont la température est à mesurer, en ce que la sonde comporte en son intérieur un circuit de mesure (81, 82) relié à ce capteur (18) et fournissant sous forme d'une donnée digitale une indication de la température du capteur (18), une unité logique (80) de commande électronique reliée au circuit de mesure (81, 82) pour le commander et en recevoir ladite donnée digitale de température, une mémoire (100) pour retenir ladite donnée digitale, une alimentation autonome (106), et des contacts extérieurs (21), et en ce que le logement (25) de la calculatrice présente des contacts électriques (27) agencés pour coopérer avec les contacts (21) de la sonde thermométrique (15), lorsque la sonde est engagée dans le logement susdit (25), lesdits contacts du logement (27) étant reliés aux moyens de calcul et de commande (40) pour assurer le transfert à ces moyens et la mémorisation par ces moyens de la donnée digitale susmentionnée, lorsqu'après avoir sorti la sonde (15) de son logement (25) et l'avoir mise en contact avec la zone à mesurer, on remet cette sonde en place dans son logement (25).

2. Calculatrice selon la revendication 1, caractérisée en ce qu'elle comporte une base de temps (48) et un circuit de mesure du temps (47) permettant, lors de la mémorisation d'une donnée, de fournir l'indication de temps ou de date associée à cette donnée.

3. Calculatrice selon la revendication 2, caractérisée en ce qu'elle comporte des moyens de commande (32) en dehors du clavier (7, 8), normalement inaccessibles, et destinés à permettre une remise à l'heure et à la date, notamment en cas de changement des batteries d'alimentation.

4. Calculatrice selon l'une des revendications précédentes, caractérisée en ce qu'elle comporte des moyens de commande (32) en dehors du clavier (7, 8), normalement inaccessibles, et permettant d'accéder à la mémoire (43, 45) au moins pour lire les données mémorisées.

5. Calculatrice selon l'une des revendications précédentes, caractérisée en ce qu'elle comporte des moyens de connexion externe (32) destinés à être reliés à un appareil de contrôle (55).

6. Calculatrice selon la revendication 5, caractérisée en ce que les moyens de calcul et de commande (40) sont agencés pour fournir à l'appareil de contrôle (55), via lesdites connexions (32), les données mémorisées concernant le cycle de la personne intéressée.

7. Calculatrice selon la revendication 1, caractérisée en ce que le capteur (18) de la sonde (15) consiste en un élément à impédance variable (85) dont la valeur dépend de la température et en ce que le circuit de mesure (81, 82) comprend un premier circuit oscillant (81) incluant ledit élément (85) à impédance variable, et dont la fréquence dépend de la valeur de cette impédance, un deuxième circuit oscillant (82) fournissant une fréquence de référence et des moyens de comptage (90) de la première fréquence commandés par un signal dépendant de la fréquence de référence et fournissant une donnée digitale correspondant à ladite température.

8. Calculatrice selon la revendication 7, caractérisée en ce que le deuxième circuit oscillant (82) est semblable au premier circuit oscillant (81) et voisin de lui, à l'exception du capteur (18, 85), de manière que si la fréquence du premier oscillateur (81), fonction de la température du capteur (18), est en outre influencée par une dérive d'alimentation ou une dérive thermique, la fréquence du deuxième oscillateur (82) est alors influencée dans le même sens par la même dérive, de sorte que finalement lors du comptage l'effet de cette dérive est pratiquement annulé.

9. Calculatrice selon l'une des revendications précédentes, caractérisée en ce que la sonde comporte des moyens de temporisation (96, 107) agencés de telle sorte que la mesure soit effectuée un certain temps après que la sonde (15) soit sortie du logement (25), de manière à permettre à l'utilisatrice de placer la sonde (15), et afin que le capteur (18) puisse s'équilibrer thermiquement avec la zone à mesurer.

10. Calculatrice selon l'une des revendications précédentes, caractérisée en ce que la sonde comporte un détecteur d'humidité (19) voisin du capteur thermométrique (18) et fournissant à l'unité de commande (80) un signal indiquant que la sonde (15) est en contact avec une muqueuse, l'unité logique de commande (80) de la sonde (15) étant agencée pour que l'acquisition de la donnée de température dépende de la présence du signal fourni par ledit détecteur d'humidité (19).

11. Calculatrice selon l'une des revendications précédentes, caractérisée en ce que la sonde (15) comporte une mémoire (100) et un comparateur (101), en ce que le circuit de mesure (81, 82), lors du fonctionnement, fournit périodiquement ladite donnée digitale de température et en ce que le comparateur (101) compare cette donnée avec le contenu de la mémoire (100) et, au cas où cette donnée est supérieure au contenu, commande l'inscription de la donnée dans la mémoire (100) de manière que la sonde (15) fonctionne comme un thermomètre à maximum.

12. Calculatrice selon l'une des revendications précédentes, caractérisée en ce que la sonde comporte une alimentation autonome rechargeable d'une autonomie suffisante pour effectuer une mesure, et destinée à être rechargée à partir de la calculatrice par le moyen de connecteurs (104, 105).

**Patentansprüche**

1. Taschenrechner für die Familienplanung, bestehend aus einem Gehäuse (1), einer Temperaturmessonde (15), einer Steuer-Tastatur (7, 8), einer Anzeige (4, 5), Mittel zum Rechnen und elektronischen Steuern (40) und Speicher (42, 43, 45), die zur Anwendung eines Verfahrens für die Familienplanung eingerichtet sind, dadurch gekennzeichnet, dass er eine Aufnahmeschale (25) aufweist, um vorübergehend die Temperaturmessonde (15) aufzunehmen, dass die Temperaturmessonde (15) vom Rechner elektrisch unabhängig ist, dass die Sonde an einem Ende einen Temperaturmessfühler (18) aufweist, der dazu bestimmt ist, mit der Zone, deren Temperatur zu messen ist, in Kontakt gebracht zu werden, dass die Sonde in ihrem Inneren eine Messschaltung (81, 82), die mit dem Fühler (18) in Verbindung steht und eine digitale Angabe der Temperatur des Fühlers (18) liefert, eine logische Einheit (8), die zur elektronischen Steuerung mit der Messschaltung (81, 82) verbunden ist um sie zu steuern und die besagte digitale Temperaturangabe aufzunehmen, einen Speicher (100) um besagte digitale Angable aufzubewahren, eine autonome Speisung (106) und externe Kontakte (21) aufweist, und dass die Aufnahmeschale (25) des Rechners elektrische Kontakte (27) aufweist, die mit den Kontakten (21) der Temperaturmessonde (15) zusammenwirken, sobald die Sonde in der vorgenannten Aufnahmeschale (25) eingelegt ist, wobei die besagten Kontakte der Aufnahmeschale (25) mit den Rechen- und Steuermitteln (40) verbunden sind, um die Uebergabe der vorgenannten digitalen Angabe an diese Mittel und die Speicherung durch diese Mittel sicherzustellen, wenn die Sonde (15) nach dem Herausnehmen aus ihrer Aufnahmeschale (25) und der Herstellung eines Kontaktes mit der zu messenden Zone wieder an ihren Platz in der Aufnahmeschale (24) zurückgelegt wird.

2. Rechner nach Anspruch 1, dadurch gekennzeichnet, dass er eine Zeitbasis (48) und eine Zeitmessschaltung (47) aufweist, die es gestatten, nach der Speicherung einer Angabe eine dieser Angabe entsprechenden Zeit- oder Datumsangabe zu liefern.

3. Rechner nach Anspruch 2, dadurch gekennzeichnet, dass er ausser der Tastatur (7, 8) Steuermittl (32) enthält, die normalerweise unzugänglich und dazu bestimmt sind, eine Zeit- und Datumseinsetzung zu ermöglichen, insbesondere im Falle eines Wechsels der Speisebatterien.

4. Rechner nach einer der vorhergehenden Ansprüche, dadurch gekennzeichnet, dass er ausser der Tastatur (7, 8) Steuermittel (32) enthält, die normalerweise unzugänglich sind und den Zugriff zum Speicher (43, 45) wenigstens zum Lesen der gespeicherten Grössen gestatten.

5. Rechner nach einem der vorhergehenden Ansprüche, dadurch gekennzeichnet, dass er externe Verbindungsstecker aufweist, die dazu bestimmt sind, mit einer Kontrollvorrichtung (55) die Verbindung herzustellen.

6. Rechner nach Anspruch 5, dadurch gekennzeichnet, dass die Rechen- und Steuermittel (40) darauf eingerichtet sind, der Kontrollvorrichtung über die besagten Steckverbindungen (32) gespeicherte Grössen zu liefern, die den Zyklus der interessierten Person betreffen.

7. Rechner nach Anspruch 1, dadurch gekennzeichnet, dass der Fühler (18) der Sonde (18) aus einem Element (85) mit veränderlicher Impedanz besteht, deren Wert von der Temperatur abhängt, und dass die Messschaltung (81, 82) eine erste Oszillatorschaltung (81) enthält, die das besagte Element (85) mit veränderlicher Impedanz einschliesst und deren Frequenz vom Wert dieser Impedanz abhängt, dass eine zweite Oszillatorschaltung (82) eine Bezugsfrequenz liefert und zum Zählen der ersten Frequenz Mittel (90) von einem Signal, das von der Bezugsfrequenz abhängig ist, gesteuert werden und eine der vorgenannten Temperatur entsprechende digitale Angabe liefern.

8. Rechner nach Anspruch 7, dadurch gekennzeichnet, dass die zweite Oszillatorschaltung (82) mit der ersten Oszillatorschaltung (81) mit Ausnahme des Fühlers (18, 85) ähnlich und benachbart ist, derart, dass, wenn die Frequenz, die eine Funktion der Temperatur des Fühlers (18) ist, ausserdem noch durch Speisungsschwankungen oder eine Temperaturdrift beeinflusst wird, die Frequenz des zweiten Oszillators (82) dann durch dieselbe Drift beeinflusst wird, derart, dass schliesslich bei der Zählung der Effekt dieser Drift praktisch annuliert wird.

9. Rechner nach einem der vorausgehenden Ansprüche, dadurch gekennzeichnet, dass die Sonde Verzögerungsmittel (96, 107) aufweist, die derart eingerichtet sind, dass die Messungeinige Zeit nach dem Herausnehmen der Sonde (15) aus der Aufnahmeschale (25) ausgeführt wird, um es der Benützerin zu gestatten, die Sonde (15) zu plazieren und der Fühler (18) sich thermisch der zu messenden Zone angleichen kann.

10. Rechner nach einem der vorhergehenden Ansprüche, dadurch gekennzeichnet, dass die Sonde einen Feuchtigkeitsmesser (19) nahe beim Temperaturmessfühler (18) aufweist, und der an die Steuereinheit (80) ein Signal liefert, das anzeigt, dass die Sonde (15) mit einer Schleimhaut in Kontakt ist und die logische Steuereinheit (80) darauf eingerichtet ist, dass die Aufnahme der Temperaturangabe vom Vorhandensein eines Signals anhängt, das vom vorgenannten Feuchtigkeitsfühler (19) geliefert wird.

11. Rechner nach einem der vorhergehenden Ansprüche, dadurch gekennzeichnet, dass die Sonde (15) einen Speicher (100) und einen Komparator (101) aufweist, und dass der Messkreis (81, 82) bei seiner Funktion periodisch die besagte digitale Angabe der Temperatur liefert und dass der Komparator (101) diese Grösse mit dem Inhalt des Speichers (100) vergleicht und im Falle diese Angabe höher als der Inhalt ist, das Einschreiben der Angabe in den Speicher (100) steuert, derart, dass die Sonde (15) wie ein Maximumthermometer funktioniert.

12. Rechner nach einem der vorhergehenden Ansprüche, dadurch gekennzeichnet, dass die Sonde eine autonome, wiederaufladbare Speisung aufweist, die ausreichend ist, um eine Messung durchzuführen und geeignet ist, aus dem Rechner mittels der Steckverbindungen (104, 105) wieder aufgeladen zu werden.

**Claims**

1. A pocket calculator for planning parenthood, comprising a casing (1), a thermometer probe (15), a control keyboard (7, 8), a display panel (4, 5), electronic control and data processing means (40) and memories (42, 43, 45), arranged for implementing a method for planning parenthood, characterized in that it comprises a receptacle (25) for temporarily accommodating the thermometer probe (15), in that the thermometer probe is electrically independent from the calculator, in that the probe is provided at one end with a temperature sensor (18) designed to be applied to the region where the temperature is measured, in that the probe contains inside a measuring circuit (81, 82) connected to the sensor (18) and generating digital data representative of the sensor (18) temperature, a logical electronic control unit (80) connected to the measuring circuit (81, 82) for controlling it and for receiving from it said digital temperature data, an autonomous power supply unit (106) and external contacts (21), in that the receptacle (25) of the calculator is provided with electrical contacts (27) designed to be connected to contacts (21) of the thermometer probe (15) when the thermometer probe is accommodated in said receptacle (25), said contacts (27) of the receptacle being connected to the control and data processing means (40) for delivering to these means and memorizing by them said digital data when the probe is replaced in its receptacle (25) after having been taken out therefrom and applied to the region where the temperature is measured.

2. A calculator according to claim 1, characterized in that it comprises a clock (48) and a time measuring circuit (47) for the purpose of recording the time or date at which temperature data are memorized.

3. A calculator according to claim 2, characterized in that it comprises control means (32) located outside the keyboard (7, 8) and normally not accessible, for resetting the time or the date, in particular after changing the power supply batteries.

4. A calculator according to any one of the preceding claims, characterised in that it comprises control means (32) located outside the keyboard (7, 8) and normally not accessible, which give access to the memory (43, 45) and permit at least the readout of the data stored in said memory.

5. A calculator according to any one of the preceding claims, characterized in that it comprises outer connection means (32) for connection to a control apparatus (55).

6. A calculator according to claim 5, characterized in that the data control and processing means (40) are devised to deliver to the control apparatus (55), through said connection means (32), the data related to the menstrual cycle of the user.

7. A calculator according to claim 1, characterized in that the sensor (18) of the probe (15) consists in an element (85) of variable impedance which is temperature dependant, in that the measuring circuit (81, 82) comprises a first oscillating circuit (81) of which said variable impedance element (85) is a component and the oscillation frequency of which is dependant upon the value of said impedance, a second oscillating circuit (82) delivering a reference frequency, and counter means (90) for measuring the frequency of the first oscillating circuit, said counter means being controlled by a signal depending upon the reference frequency, and for providing digital data representative of said temperature.

8. A calculator according to claim 7, characterized in that the second oscillating circuit (82) is located close to the first oscillating circuit (81) and is similar to this first oscillating circuit except for the sensor (18, 85), so that if the frequency of the first oscillating circuit (81) which is primarily function of the sensor (18) temperature happened to be affected by a drift due to the power supply or by a thermal drift, the frequency of the second oscillating circuit would be affected in the same manner, thereby substantially eliminating the effect of such drifts on the output of the counter means.

9. A calculator according to any one of the preceding claims, characterized in that the probe comprises timing means (96, 107) devised so that the temperature is measured a certain time after the removal of the probe (15) from the receptacle (25), thus giving time to the user to position the probe (15) and to the sensor (18) to reach a thermal equilibrium with the region where the temperature is measured.

10. A calculator according to any one of the preceding claims, characterized in that the probe comprises a humidity detector (19) located close to the temperature sensor (18), which provides the control unit (80) with a signal indicating that the probe is positioned against a mucous membrane, the logical control unit (80) of the probe (15) being devised in such a manner that the acquisition of the temperature data is contingent on the presence of a signal from the humidity detector (19).

11. A calculator according to any one of the preceding claims, characterized in that the probe (15) comprises a memory (100) and a comparator (101), in that the measuring circuit (81, 82) when operating provides at regular intervals said digital temperature data, in that the comparator (101) compares such data with the content of the memory (100), and permits the storage of said data in the memory (100) if its value is above that already stored, which results in the probe functioning in the manner of a maximum thermometer.

12. A calculator according to any one of the preceding claims, characterized in that the probe comprises an autonomous rechargeable power unit with sufficient power to perform a measure and designed to be recharged from the calculator through connecting means (104, 105).

# FIG.1

PROBABILITÉ ELEVÉE  MOYENNE  NULLE

JOUR  MOIS  ANNÉE

| $x^2$ | $\sqrt{\phantom{x}}$ | % | ON/C | OFF |
| 7 | 8 | 9 | ÷ | R |
| 4 | 5 | 6 | × | MR |
| 1 | 2 | 3 | − | M− |
| 0 | • | = | + | M+ |

# FIG.2

# FIG.3

FIG.4

FIG.5

FIG.6a

FIG.6b

FIG.7

# FIG.8

# FIG.9